# EUROPEAN PATENT APPLICATION

(11) **EP 4 749 631 A1**
(43) Date of publication of application: **27.05.2026**
(21) Application number: 25216948.7
(22) Date of filing: 19.11.2025
(51) Int. Cl.: G16H 10/60

(54) **INFORMATION PROCESSING SYSTEM, METHOD FOR PROCESSING INFORMATION, COMPUTER PROGRAM, AND RECORDING MEDIUM**

(30) Priority: 26.11.2024 JP 2024205454
(71) Applicant: Canon Kabushiki Kaisha, Tokyo, 146-8501 (JP)
(72) Inventor: YAMASHITA, Kouki, Tokyo, 146-8501 (JP); MIZOBE, Hideaki, Tokyo, 146-8501 (JP)
(74) Representative: Canon Europe Limited

(57) **Abstract**

According to the present disclosure, an information processing system includes a data acquisition unit (103a), a feature acquisition unit (103c), and a similarity acquisition unit (103d). The data acquisition unit (103a) is configured to acquire first medical data including a plurality of items or different types of data relating to a subject. The feature acquisition unit (103c) is configured to acquire features representing characteristics of medical data. The feature acquisition unit (103c) acquires at least a subset of the features by inputting the medical data included in the first medical data into at least one of an encoder of a large language model and an encoder of a large multimodal model. The similarity acquisition unit (103d) is configured to acquire similarity between the features and a feature of another piece of the medical data.

## Description

### TECHNICAL FIELD

The present disclosure relates to an information processing system, a method for processing information, a computer program, and a recording medium.

### BACKGROUND

A technique for searching for similar data is known in which data such as text or images is converted into a numerical representation to perform the search.

For example, Japanese Patent Laid-Open Publication No. 2008-027131 discloses a technique for calculating a feature from a partial image in a document image and searching for another document having a similar feature.

### SUMMARY

the method described in Japanese Patent Laid-Open Publication No. 2008-027131 does not take into account a case where similar data is to be searched using a plurality of types of data including text, or a case where similar data is to be searched in consideration of user's intention. Accordingly, it may be difficult to appropriately calculate the similarity in some cases.

The present disclosure is aimed at providing an information processing system, a method for processing information, a computer program, and a recording medium that enable appropriate calculation of similarity.

The present disclosure in its first aspect provides an information processing system as specified in claim 1. Optional features are specified in claims 2 to 18. Optional features of the first aspect are also optional features of the other aspects of the present disclosure.

The present disclosure in its second aspect provides a method for processing information as specified in claim 19. Optional features are specified in claim 20.

The present disclosure in its third aspect provides a computer program comprising instructions which, when the program is executed by a computer, cause the computer to carry out the method of the second aspect.

The present disclosure in its fourth aspect provides a computer-readable data carrier having stored thereon the computer program of the third aspect.

Features of the present disclosure will become apparent from the following description of embodiments with reference to the attached drawings. The following description of embodiments is described by way of example.

### BRIEF DESCRIPTION OF THE DRAWINGS

Fig. 1 illustrates an example of a functional configuration of an information processing system according to a first embodiment.
Fig. 2 is a flowchart of processes executed by the information processing system according to the first embodiment.
Fig. 3 is a flowchart of processes of storing in a database executed by an information processing system according to modification 2 of the first embodiment.
Fig. 4 is a flowchart of processes executed by the information processing system according to modification 2 of the first embodiment.
Fig. 5 is a flowchart of processes executed by an information processing system according to modification 3 of the first embodiment.
Fig. 6 is a flowchart of processes executed by an information processing system according to modification 5 of the first embodiment.
Fig. 7 is a configuration example of medical record information handled by the information processing system.
Fig. 8 is an example of a SOAP note handled by the information processing system.
Fig. 9 is a configuration example of the database handled by the information processing system.
Fig. 10 illustrates an example of a functional configuration of an information processing system according to a second embodiment.
Fig. 11 is a flowchart of processes executed by the information processing system according to the second embodiment.

### DESCRIPTION OF THE EMBODIMENTS

Hereinafter, embodiments of the present disclosure will be described with reference to the drawings. It should be understood that the present disclosure is not limited to the embodiments below.

In the drawings described below, like elements are denoted by the same reference numerals, and redundant explanation may be omitted or simplified.

### First Embodiment

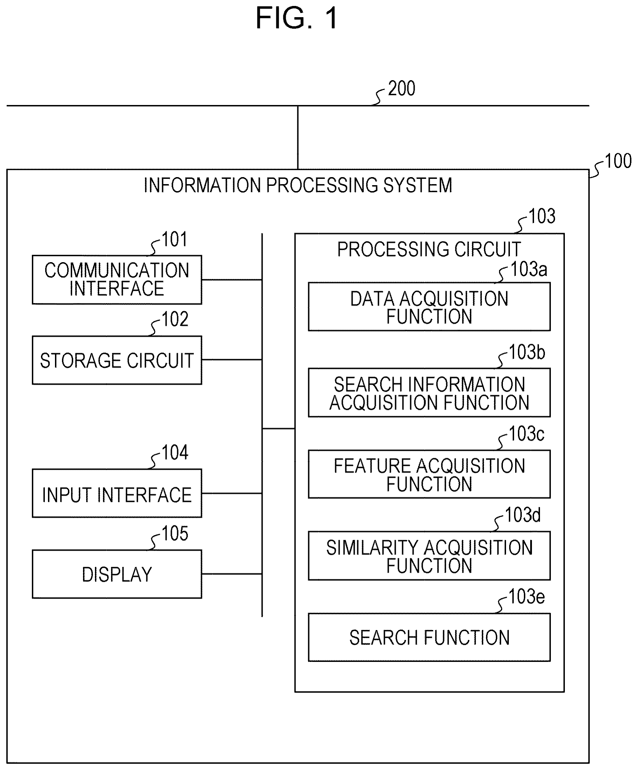

Fig. 1 illustrates an example of a functional configuration of an information processing system 100 according to a first embodiment. The information processing system 100 is configured to acquire medical data and execute information processing on the medical data. Herein, target data is not limited to the medical data. The target data may be content data including the above-described data.

Herein, the medical data relating to the present embodiment is data in which a plurality of data items coexist. The data items may include different media forms (numerical values, categories, text, sounds, still images, moving images, and so forth). For example, medical record information relating to a single patient managed by an electronic medical record is an example of the medical data including the plurality of items relating to the present embodiment.

Specifically, the medical record information that is the medical data may include data items in numerical form such as the age, height, weight, and blood test data of the patient (subject). The medical record information as the medical data may also include data items in category form such as the gender (male, female, other), the presence/absence of smoking history, the presence/absence of family disease history, and the presence/absence of consent to diagnosis and hospitalization of the patient (subject).

Furthermore, the medical record information may also include the name of the patient, the entirety of a note by a SOAP (Subjective, Objective, Assessment, and Plan) method, (hereinafter, referred to as a SOAP note) in a medical care and nursing care record, diagnosis, and link information to external systems (such as a department system and a web site). The medical record information may also include data items in text form such as sentences in an examination report that can be acquired based on the link information.

The medical record information may also include data items in image form such as scanned images of a referral and a certificate of consent, examination images such as an X-ray image and a computerized tomography (CT) image that can be acquired based on the above-described link information, and key images in an examination report. The medical record information may also include, for example, data items in waveform such as an electrocardiogram, data items in sound form such as a body sound acquired by a digital stethoscope, and data items in moving image form and coordinate form relating to walking data in rehabilitation.

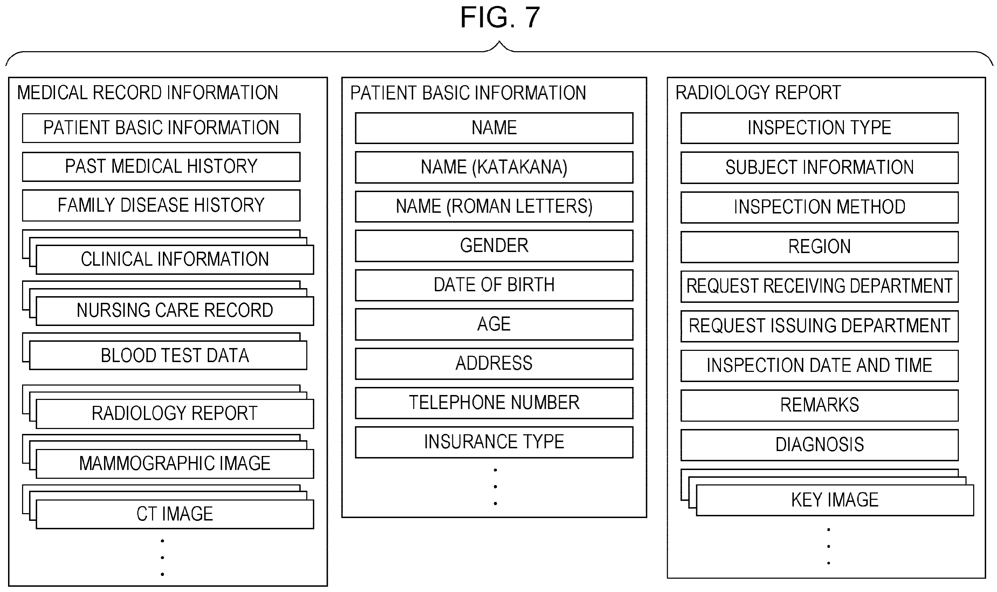

As a specific example, exemplary data items included in the medical record information are illustrated in Fig. 7. Digital imaging and communications in medicine (DICOM) image data relating to a single examination or single imaging managed by a picture archiving and communication system (PACS) is an example of the medical data including data corresponding to a plurality of data items relating to the present embodiment. Specifically, the DICOM image data may include data items in numerical form such as information about imaging positions and pixel configurations. Furthermore, the DICOM image data may also include data items in text form such as the name of an imaging apparatus, the name of an institution, the name of the patient, identifiers of the examination, and the name of the region. Furthermore, the DICOM image data may also include data items in image form such as image data and overlay data. Furthermore, the DICOM image data may also include data items in specific form to be utilized by only a subset of DICOM image viewers. Depending on the imaging apparatus or the like that outputs the DICOM image data, the image data may be two-dimensional or three-dimensional images or two-dimensional or three-dimensional moving images.

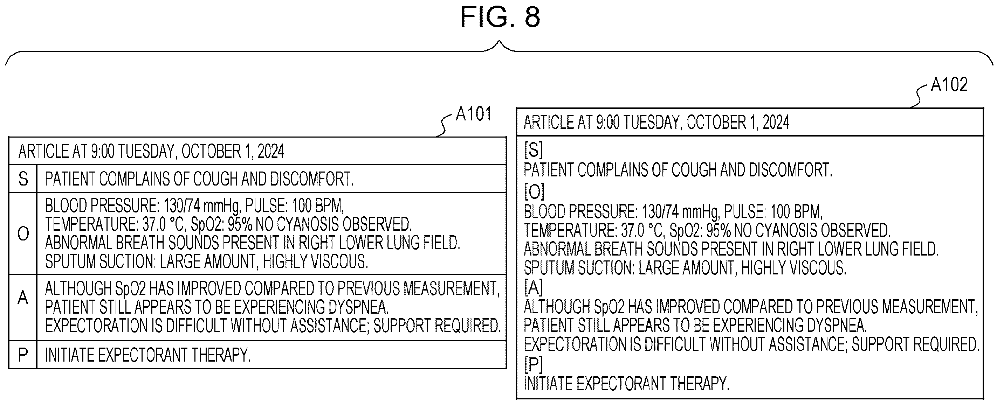

Furthermore, for example, the SOAP note in the medical care and nursing care record can be regarded as an example of the medical data in which a plurality of data items relating to the present embodiment coexist. The SOAP note is medical data including four data items in text form, that is, "a note about subjective information", "a note about objective information", "a note about assessment", and "a note about a treatment plan". A data item group included in the SOAP note may be recorded as a single data item depending on use of the electronic medical record. As a specific example, an example of the SOAP note is illustrated in Fig. 8. A SOAP note A101 has a structure including four data item groups. A SOAP note A102 is an example that is not structured and has only one data item. In the present embodiment, for ease of understanding, the SOAP note is assumed to include four data items. When the SOAP note is not structured, processing of division into four data items may be used or the entirety of the SOAP note may be handled as a single item.

The information processing system 100 may be realized by, for example, a computer device such as a server or a workstation. The information processing system 100 may display a result of information processing, store the result of the information processing, and output the result of the information processing to an external apparatus. For example, the information processing system 100 may search other medical data similar to arbitrary medical data. Link information and general information corresponding to a subset of similar medical data groups that are the results of the search may be displayed by a display 105 or a display apparatus (not illustrated) connected to the information processing system 100.

The information processing system 100 may be communicably connected to a medical data management apparatus (not illustrated) to acquire the medical data via a network 200 or, for example, a communication cable (not illustrated) or a communication circuit (not illustrated). The medical data management apparatus may be, as one of elements of the information processing system 100, integrated with the information processing system 100.

The medical data management apparatus is a database apparatus configured to be able to store the medical data and transmit and receive the medical data to and from another apparatus communicable with the medical data management apparatus such as the information processing system 100. The medical data management apparatus may receive a search query for the medical data from the other apparatus communicable with the medical data management apparatus such as the information processing system 100 and transmit the search result. Specifically, for example, the medical data management apparatus may receive a query using a structured query language (SQL), in which a numerical value or a character string included in the data items of the medical data is designated as a search condition, and may transmit medical data groups matching the condition, and identifier groups of the medical data groups, and the like.

The medical data management apparatus may relate features in vector (numerical value group) form indicating the characteristics of at least a subset of the data item groups included in the medical data to the stored medical data and store the features. Furthermore, the medical data management apparatus may receive a query in which a feature is designated as a search condition and transmit the medical data groups or the identifier groups of the medical data groups matching the feature or related to a similar feature.

The information processing system 100 may be connected to another apparatus so as to acquire the features in vector form indicating the characteristics of at least a subset of the data item groups included in the medical data. For example, the information processing system 100 may be communicably connected to a feature acquisition apparatus (not illustrated) via the network 200 or, for example, a communication cable (not illustrated) or a communication circuit (not illustrated). The feature acquisition apparatus may be, as one of the elements of the information processing system 100, integrated with the information processing system 100.

The feature acquisition apparatus is configured to be able to acquire at least a subset of the data item groups included in the medical data, calculate the features based on the acquired data item groups, and transmit the calculated features to another apparatus communicable with the feature acquisition apparatus such as the information processing system 100. The feature acquisition apparatus includes one or more encoders configured to calculate, when data corresponding to one or more data items is input, the feature in vector form representing a characteristic of the data corresponding to the data items. As an encoder, an encoder included in a large language model or a large multimodal model can be used.

The encoder included in the large language model is a text encoder into which a text prompt in text form can be input.

For example, "Large Language Model Meta AI (Llama)" of Meta Platforms, Inc. is an example of the large language model. An encoder included in the large multimodal model is any of encoders for different media forms that can receive prompts in different media forms (numerical value encoder, category encoder, text encoder, sound encoder, image (still and moving) encoder).

For example, "Contrastive Language-Image Pre-Training (CLIP)" of OpenAl is an example of the large multimodal model. The encoder receives data in corresponding media form and outputs the feature in vector form representing the characteristic such as content or a concept relating to the data. Data in numerical form or category form can also be input into the text encoder when such data is changed into a character string (text).

As the encoder, part of a neural network configured to execute a predetermined image processing task such as image classification, image generation, image segmentation, metric learning, or contrastive learning may be utilized. That is, an intermediate feature calculated when data is input to the neural network may be regarded as the feature representing the characteristic of the data.

The encoder may be trained such that feature groups acquired by inputting similar data groups into the encoder have a high similarity while feature groups acquired by inputting dissimilar data groups have a low similarity.

Specifically, it is assumed that, for example, out of images A to C, only image C is not similar to the other two images. At this time, regarding the features A to C acquired by inputting the images into the encoder, the encoder may be trained such that the similarity between the features A and B is high, and similarities between the features A and C and the similarity between B and C are low.

In other words, the encoder is characterized as follows: the encoder is trained such that the similarity of the feature groups to be output in response to input of inspection data groups in which the characteristics of predetermined data item groups are similar is higher than the similarity of the feature groups to be output in response to input of dissimilar inspection data groups.

This training may be achieved by fine-tuning a trained encoder. For the fine-tuning, a general learning method such as supervised learning, reinforcement learning, training only the output layer, or low-rank adaptation (LoRA) can be used.

Here, in calculating the similarity between the features using a similarity acquisition function, as the number of dimensions of the feature in vector form increases, the difference in distance between the vectors decreases. This causes a problem in that the identification of the similarity becomes difficult. Thus, the similarity acquisition function may apply a dimensionality reduction technique to the feature as preprocessing of calculation of the similarity of the feature. Examples of the dimensionality reduction technique include, for example, a principal component analysis (PCA), t-stochastic neighbor embedding (t-SNE), and uniform manifold approximation and projection (UMAP).

There is a method in which the dimensionality is reduced by leaving only dimensions useful for evaluation of the similarity between the features and removing values of the other dimensions based on a statistical analysis performed on the feature groups, using the similarity acquisition function, acquired by inputting similar data groups and dissimilar data groups into the encoder. Specifically, for example, the similarity acquisition function may remove the values of the dimensions with large variance in the feature groups acquired by inputting the similar data groups into the encoder.

Whether a data group is defined as similar or dissimilar may differ depending on the viewpoints of a user. For example, it is assumed that, in an image group, the images can be grouped into a dark image group and a bright image group based on the brightness and grouped into an image group illustrating a human upper body and an image group of a lower body based on imaged regions.

In a certain viewpoint, it can be said that each group is similar. However, for example, it is not necessarily the case that the upper body (or lower body) is imaged in all the images in the dark image group. There is a possibility that images dissimilar from the viewpoint of the imaged region are gathered in the dark image group.

Accordingly, in executing similar data search, the characteristic to evaluate the similarity can be changed in accordance with user's intention. That is, in accordance with the user's intention, the dimensions to be removed in the above-described dimensionality reduction can be changed and a combination of data for evaluation of the similarity can be changed. In the present embodiment, to allow similar data search to be executed as intended by the user, the above-described changes can be performed in accordance with search information, which will be described later.

Fig. 1 illustrates an example of the configuration of the information processing system 100 according to the present embodiment. As illustrated in Fig. 1, the information processing system 100 includes a communication interface 101, a storage circuit 102, a processing circuit 103, an input interface 104, and the display 105. The information processing system 100 can be communicably connected to the network 200 via the communication interface 101.

The communication interface 101 is configured to communicate with another apparatus for the medical data, search results, and the like. The communication interface 101 is realized by, for example, a network communication interface such as a network adaptor or a network interface controller (NIC). Alternatively, the communication interface 101 may be realized by a device connection interface such as a universal serial bus (USB), peripheral component interconnect express (PCI Express), serial advanced technology attachment (SATA, serial ATA), or M.2.

The storage circuit 102 is configured to store various types of data and various types of programs to be used for processes executed by the information processing system 100 according to the present embodiment. Specifically, the storage circuit 102 is connected to the processing circuit 103 and operated under control of the processing circuit 103. The storage circuit 102 also has the function of work memory that temporarily stores various types of data to be used for processes executed by the processing circuit 103. The storage circuit 102 is realized by, for example, a semiconductor memory device such as a random-access memory (RAM) or a flash memory, a hard disc drive, an optical disc, or the like.

The processing circuit 103 is configured to control operation of the above-described components of the information processing system 100. For example, the processing circuit 103 performs the various types of processes in accordance with instructions accepted from the user via the input interface 104 connected to the information processing system 100. Alternatively, for example, the processing circuit 103 may perform the various types of processes in accordance with instructions accepted from the user via the communication interface 101. The processing circuit 103 is realized by, for example, a central processing unit (CPU).

The processing circuit 103 includes, for example, a data acquisition function 103a that realizes a data acquisition unit configured to acquire the medical data and a search information acquisition function 103b that realizes a search information acquisition unit configured to acquire the search information. The processing circuit 103 also includes a feature acquisition function 103c that realizes a feature acquisition unit configured to acquire the feature and a similarity acquisition function 103d that realizes a similarity acquisition unit configured to acquire the similarity between pieces of medical data. The processing circuit 103 also includes a search function 103e that realizes a search unit configured to acquire a search result of similar medical data.

When the information processing system 100 has the above-described functional configuration, the information processing system 100 can adequately acquire the similarity to the medical data intended by the user. When the information processing system 100 performs the search using the similarity, the information processing system 100 can acquire the search result in conformity with user's search intention.

Here, for example, each of the processing functions realized by the corresponding elements of the processing circuit 103 illustrated in Fig. 1 is stored in the storage circuit 102 in the form of a program executable by the computer.

The processing circuit 103 reads the programs from the storage circuit 102 and executes the read programs, thereby realizing the functions corresponding to the programs. That is, the processing circuit 103 having read the programs has the functions realized by the data acquisition function 103a, the search information acquisition function 103b, the feature acquisition function 103c, the similarity acquisition function 103d, and the search function 103e. Specifically, the information processing system 100 according to the present embodiment has the data acquisition function 103a configured to acquire first medical data that relates to the subject and includes a plurality of items. The information processing system 100 also has the search information acquisition function 103b configured to acquire the search information that is information for searching medical data (second medical data) similar to the first medical data. In addition, the information processing system 100 has the feature acquisition function 103c configured to acquire, based on the search information, the feature representing the characteristic of the medical data. The feature acquisition function 103c is characterized in that the feature acquisition function 103c is configured to acquire at least a subset of the features by inputting medical data included in the first medical data into at least one of the encoder of the large language model and the encoder of the large multimodal model. The information processing system 100 is characterized in that the information processing system 100 also has the similarity acquisition function 103d configured to acquire the similarity between the feature and the feature of another piece of medical data. The information processing system 100 also has the search function 103e configured to acquire the search result of the medical data (the second medical data) similar to the first medical data based on the similarity. Here, the search information includes the user's search intention and changes the feature acquired via at least one of the encoder of the large language model and the encoder of the large multimodal model using the similarity acquisition function 103d. Alternatively, the search information includes the user's search intention and acquires the feature from the first feature acquired via at least one of the encoder of the large language model and the encoder of the large multimodal model using the similarity acquisition function. As described above, the feature acquisition function 103c may include a different apparatus. When the calculation of the feature is executed by the different apparatus, the feature acquisition function 103c may acquire the calculated feature. Alternatively, the information processing system 100 may have the function of the feature acquisition apparatus.

The input interface 104 is configured to accept input operation of various types of instructions and various types of information from the user into the information processing system 100.

Specifically, the input interface 104 is connected to the processing circuit 103, converts the input information received from the user into an electric signal, and transmits the signal to the processing circuit 103. For example, the input interface 104 is realized by a trackball, a switch button, a mouse, a keyboard, or a touch pad that allows, when an operation surface thereof is touched, input operation to be performed. Alternatively, the input interface 104 may be realized by a touch screen formed by integrating a display surface and the touch pad with each other, a contactless input interface using an optical sensor, a voice input interface, or the like.

The input interface 104 is not limited to an interface including a physical operation component such as a mouse or keyboard. For example, examples of the input interface 104 include an electric signal processing circuit configured to receive an electric signal corresponding to input operation from an external input device provided separately from the information processing system 100 and transmit this electric signal to the processing circuit 103.

The display 105 is configured to display, with a graphical user interface (GUI), various types of data such as the data based on the medical data and the search result processed by the information processing system 100. Specifically, the display 105 is connected to the processing circuit 103 and displays the various types of data received from the processing circuit 103. For example, the display 105 displays the data items in the text form and the data items in image form based on the medical data and also displays the link information and general information corresponding to a subset of the similar medical data groups that are the search results. Specifically, the display 105 is realized by, for example, a liquid crystal display monitor, a cathode ray tube (CRT) monitor, a touch panel, or the like.

The above description is dedicated to the example of the functional configuration of the information processing system 100 according to the present embodiment.

Hereinafter, an example of acquiring the medical data highly similar to the user's search intention is described. This acquisition is performed in a search process, executed by the information processing system 100 according to the present embodiment, of the medical data similar to the first medical data including the plurality of data items. In other words, an example unlikely to include medical data having low relevance to the user's search intention is presented.

In the present embodiment, for ease of description, the medical data is the inspection data of the patient in the example. Specifically, the medical data is the SOAP note including four data items in text form described in the medical record information of the electronic medical record. Furthermore, the medical data management apparatus is an electronic medical record system that is a database managing the SOAP note.

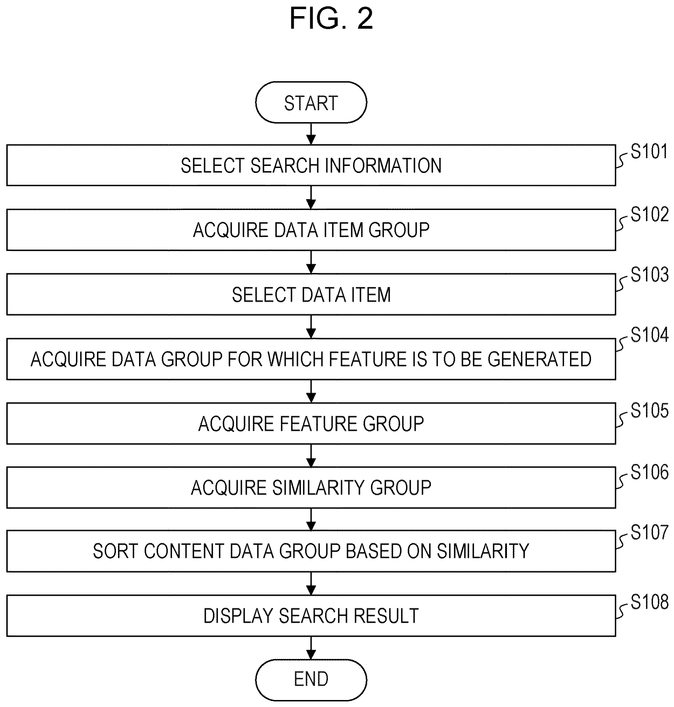

Hereinafter, an example of the flow of the processes executed by the information processing system 100 according to the present embodiment is described with reference to a flowchart illustrated in Fig. 2. Order of steps and order of procedures in the steps to be described herein may be changed without causing a contradiction.

In the following description, for ease of understanding, it is assumed that the user operates the input interface 104 and selects a single SOAP note from the electronic medical record system. The user is currently editing the selected SOAP note. It is also assumed that the user is performing the similar data search on the electronic medical record system to search for the other SOAP note similar to the SOAP note for reference. In the following description, the selected SOAP note is referred to as "search condition data", and SOAP notes included in the electronic medical record system other than the "search condition data" are referred to as "searched data".

In step S101, the search information acquisition function 103b acquires, as the search information, a search purpose selected by the user via the GUI displayed by the display 105.

It is assumed that, in the description of the present embodiment, the purpose is selectable from three examples of the search purpose, "assessment", "planning", and "none in particular".

In step S102, the data acquisition function 103a acquires the first medical data including the plurality of items. Specifically, the data acquisition function 103a acquires all the SOAP note groups stored in the electronic medical record system. The SOAP note is the medical data including the four data items in text form, that is, "a note about subjective information", "a note about objective information", "a note about assessment", and "a note about a treatment plan".

In step S103, the feature acquisition function 103c selects the data items included in pieces of the medical data (SOAP notes) acquired in step S102 based on the search purpose that is the search information. For example, when the search information is "assessment", two data items, "a note about subjective information" and "a note about objective information" are selected. When the search information is "planning", two data items, "a note about objective information" and "a note about assessment" are selected. When the search information is "none in particular", all the data items, that is, four data items are selected.

In step S104, the feature acquisition function 103c executes a processing procedure for the data items based on the search information. Specifically, for a single SOAP note, the data items selected in the procedure in step S103 is applied to a template note selected based on the search information to generate a single text data piece. When this operation with the search information is executed for each SOAP note, single text data piece groups are acquired. In the following description, it is assumed that, out of description in a certain SOAP note, "a note about subjective information" is text T1, "a note about objective information" is text T2, "a note about assessment" is text T3, and "a note about a treatment plan" is text T4.

For example, when a template note to be used for the search information of "assessment" is exemplified, the template note is in a form as follows: "A note about subjective information is '<place holder P1>'. A note about objective information is '<place holder P1>‴. When actually selected data items are applied to the template note, the operation is as follows: the part <place holder P2> is substituted by text T1 and the part <place holder P2> is substituted by text T2. That is, a single text data piece, "A note about subjective information is 'text T1'. A note about objective information is 'text T2'" is generated. When the search information is "planning", a single text data piece "A note about objective information is 'text T2'. A note about assessment is 'text T3'." is generated in a similar manner. When the search information is "none in particular", a single text data piece "A note about subjective information is 'text T1'. A note about objective information is 'text T2'. A note about assessment is 'text T3'. 'A note about a treatment plan is text T4'." is generated in a similar manner. Here, examples of the generation of the simple single text data pieces are described. The template note may be changed for improvement of the performance. Furthermore, the single text data piece may be generated by simply coupling selected data items without the use of the template note.

In step S105, the feature acquisition function 103c transmits, to the feature acquisition apparatus (not illustrated), the single text data piece groups generated in the procedure in step S104. The feature acquisition apparatus inputs each of the received single text data piece groups into the encoder provided in the feature acquisition apparatus and transmits the calculated feature groups to the feature acquisition function 103c. The feature acquisition function 103c receives and acquires the feature groups. In other words, the feature acquisition function 103c is characterized in that the feature acquisition function 103c inputs a prompt in conformity with the user's search intention into the encoder for acquiring the feature in conformity with the user's search intention.

In step S106, the similarity acquisition function 103d calculates, by using the feature groups acquired in step S105, the similarity groups with the features corresponding to the search condition data and the similarity groups with the feature groups corresponding to the searched data groups. The feature is in vector form. Thus, to evaluate the similarities between two feature differences, it is sufficient that the similarities be quantified so as to be comparative.

To quantify the similarity, for example, a general technique of evaluating vector similarity such as Manhattan distance, Euclidean distance, a cosine similarity, Jaccard coefficient, or a neural network trained to calculate the similarity may be used.

In step S107, the search function 103e sorts the searched data groups corresponding to the similarity based on the magnitude of the values of the similarity calculated in step S106. Thus, even when a large amount of searched data exists, accessibility, for the user, of the SOAP note similar to the search condition data is improved. Specifically, for example, when the similarity calculated in step S106 is the Euclidean distance between the features, the similarity increases as the value of the similarity becomes close to zero. Thus, the values of the similarity are sorted in the ascending order, and the searched data groups are sorted corresponding to the order of the sorted similarity. The search function 103e may acquire a search result of similar medical data based on a value of an item included in the medical data in addition to the similarity between the features.

In step S108, the search function 103e causes the sorted searched data groups to be displayed as the search result in the GUI of the display 105. At this time, to limit the number of the SOAP notes included in the search result, the similarities from the highest to the Nth (N is a predetermined number) may be set as the search result.

When the entirety of each SOAP note of the search result is displayed in the GUI, the result is not necessarily easily reviewed due to excessive amount of information. Instead, the search function 103e may cause link information to be displayed for screen transition to the medical record information of the patient corresponding to the SOAP note of the search result.

Instead of causing the entirety of each SOAP note of the search result to be displayed in the GUI, the search function 103e may cause a summarized SOAP note using the large language model to be displayed. Instead of causing the entirety of each SOAP note of the search result to be displayed in the GUI, only a subset of the data items included in the SOAP note may be displayed. For example, specifically, when the search information is "assessment", only "a note about subjective information" and "a note about objective information" may be displayed, or only "a note about assessment" may be displayed. Instead of causing the entirety of each SOAP note of the search result to be displayed in the GUI, only first X characters (X is a predetermined number) of the note corresponding to each data item included in the SOAP note may be displayed.

From the above description, the information processing system 100 according to the present embodiment can calculate appropriate similarity in consideration of the user's search intention in the search process for the medical data similar to the medical data including the plurality of data items. Searching by the search function 103e using the similarity can reduce the likelihood of including medical data having low relevance to the user's search intention.

For example, it is assumed that the user is describing "a note about assessment" of the SOAP note and referring to "a note about assessment" of another case. In this situation, "a note about assessment" or "a note about a treatment plan" of the SOAP note has not been described. In such cases, the related-art similar data search may include data in which, similarly, "a note about assessment" or "a note about a treatment plan" has not been described may be included in the search result as the similar data. However, with the information processing system 100 according to the present embodiment, user sets the search information. Thus, for example, the similar data search can be executed using the similarity of only "a note about subjective information" and "a note about objective information". That is, the search information is based on the instructions from the user. This reduces, compared to the related-art, the likelihood of the search result including the data in which the user is not interested.

Hereinafter, modifications of the processes of the information processing system 100 according to the above-described first embodiment are described. In the following description, configurations and processes similar to those of the information processing system 100 are denoted by the same reference numerals, thereby appropriately omitting the detailed description.

### Modification 1 of First Embodiment

Although the search function 103e causes the search result to be displayed in the GUI of the display 105 in step S108 according to the first embodiment, the search result may be transmitted to an external system (not illustrated) according to modification 1. At this time, the search result may be or is not necessarily displayed in the GUI of the display 105.

In the present modification, the information processing system 100 can transmit the search result to the external system. Thus, the external system can cause the search result to be displayed by the display apparatus (not illustrated) and apply the search result to information analysis.

### Modification 2 of First Embodiment

A calculation cost of the feature acquisition in step S105 of the first embodiment is generally high, and when a large number of pieces of the searched data exist, a time taken to acquire the search result may increase. To address this, for each SOAP note stored in the electronic medical record system, the features corresponding to a plurality of search purposes may be calculated in advance before the similar data search is executed. Furthermore, the features may be associated with the SOAP notes and the search purposes and stored in a database to reduce time taken to acquire the search result. Here, the database is the medical data management apparatus (not illustrated) communicably connected to the information processing system 100. The database is a separately provided database system or a database included in the electronic medical record system.

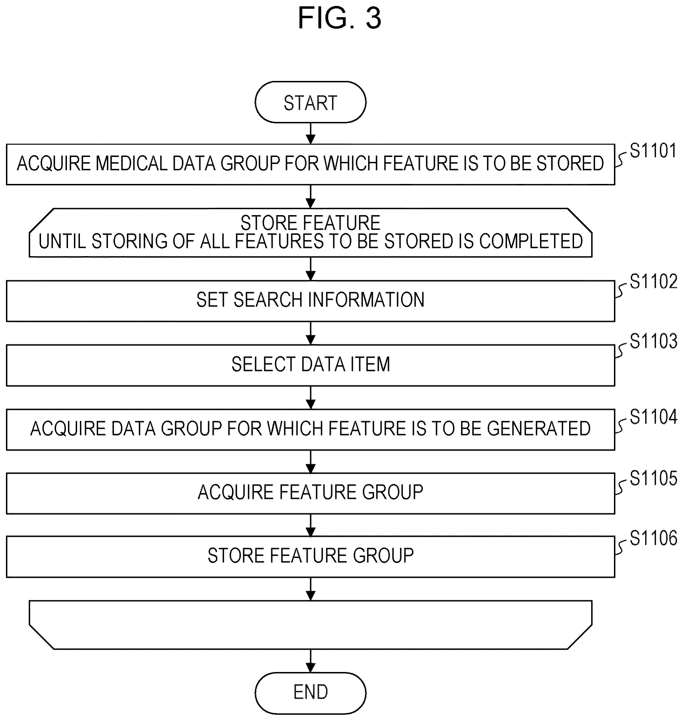

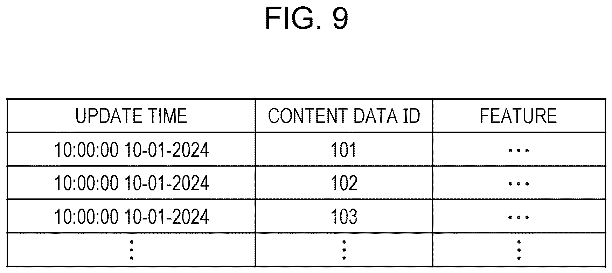

First, an example of the flow of the processes executed by the information processing system 100 in the present modification is described with reference to a flowchart illustrated in Fig. 3. These processes are executed to store, in advance, the feature of the SOAP note of the electronic medical record system in the database. Fig. 9 illustrates an example of a table of the database handled in the present modification. In the present modification, a single type of the feature is utilized in the calculation of the similarity. Thus, also a single type of the feature is managed in the table. When a plurality of types of the feature are utilized in the calculation of the similarity, the number of types of the feature to be managed in the table may be changed correspondingly. Order of steps and order of procedures in the steps to be described herein may be changed without causing a contradiction.

The flow of processes below is preferably executed in a time period during which the similar data search by the operation of the user is not executed. For example, the execution of the flow may be triggered by an instruction of an administrator of the information processing system 100, a predetermined time and date (at midnight of weekdays), a predetermined operation by the user (such as operation for finishing the edition of a SOAP note), or the like.

In step S1101, the data acquisition function 103a acquires the SOAP note groups for which the feature based on the SOAP note and the search purpose are deserved to be stored in the database. For example, when it is not the case that the feature based on any of the search purposes for a SOAP note exists in the database, the SOAP note groups are acquired. For example, when a SOAP note is updated after the existing feature of the SOAP note has been stored, the SOAP note groups are acquired. When all the features stored in the database are wanted to be updated because of, for example, a change in the feature acquisition apparatus, all the SOAP note groups are acquired.

In step S1102, the search information acquisition function 103b selects a search purpose that has not been selected even once in the flow of a series of processes (steps S1101 to S1106). When there is no search purpose that has not been selected even once, the processing ends.

In steps S1103 to S1104, processes similar to the processes in steps S101 to S104 of the first embodiment are executed.

In step S1105, a process similar to the process in step S105 of the first embodiment is executed.

In step S1106, the feature acquisition function 103c associates the acquired feature groups with the corresponding SOAP notes of the electronic medical records and the search purposes selected in step S1102 and stores the acquired feature groups in the database. The processing returns to step S1102.

From the above description, the information processing system 100 of the present modification can associate, for each of the SOAP note groups stored in the electronic medical records, the features corresponding to the search purposes with the SOAP note and store the features in the database.

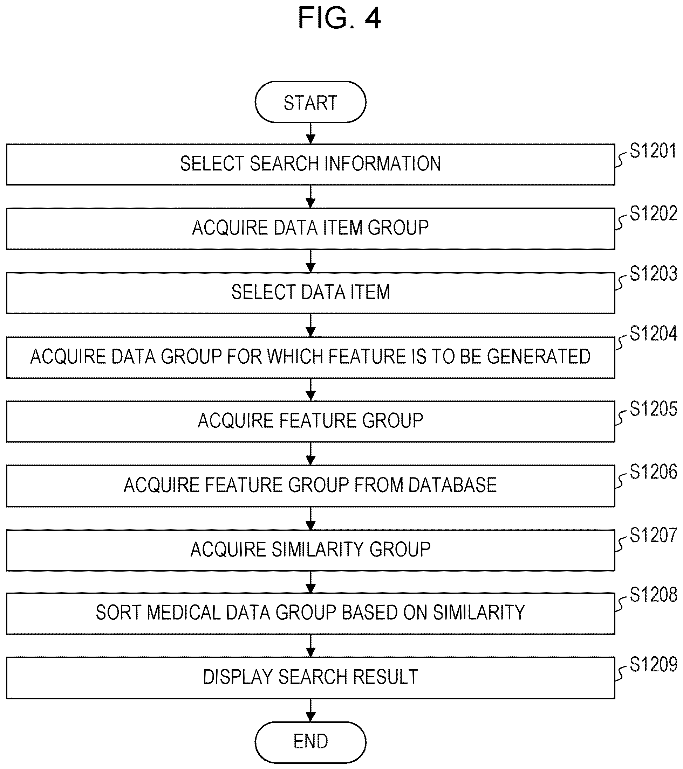

Next, as an example, the following state is described: one or more features associated with the SOAP notes in the electronic medical records have been stored in the database as a result of the execution of the above-described flow of the series of the processes (steps S1101 to S1106). An example of the flow of executing the processes of the similar data search is described with reference to a flowchart illustrated in Fig. 4. Order of steps and order of procedures in the steps to be described herein may be changed without causing a contradiction.

The flow of processes below describes an example in which, as in the first embodiment, the user operates the input interface 104 to search the similar data starting from a state in which a single SOAP note has been selected and acquired from the electronic medical record.

In step S1201, processes similar to the processes in step S101 of the first embodiment are executed.

In step S1202, the data acquisition function 103a acquires, out of the SOAP notes stored in the electronic medical record, the SOAP note groups added or updated after the last execution of step S1101 described above.

In steps S1203 and S1204, processes similar to the processes in steps S103 and S102 of the first embodiment are executed.

In step S1205, a process similar to the process in step S105 of the first embodiment is executed. Furthermore, the feature acquisition function 103c associates the acquired feature groups with the corresponding SOAP notes and the inspection purposes and stores the acquired feature groups in the database. In this way, reduction of the calculation cost of the acquisition of the feature in executing the similar data search at the next time may be tried.

In step S1206, the feature acquisition function 103c acquires feature groups that are associated with remaining SOAP note groups not having been acquired in step S1202 and the search purposes selected in step S1201, and these feature groups are stored in the database.

In step S1207, the similarity acquisition function 103d calculates the similarity by using the feature groups acquired from the feature acquisition apparatus in step S1205 and the feature groups acquired from the database in step S1206. Specifically, the similarity acquisition function 103d calculates the similarity groups with the features corresponding to the search condition data and the similarity groups with the feature groups corresponding to the searched data groups.

In steps S1208 and S1209, processes similar to the processes in steps S107 and S108 of the first embodiment are executed.

From the above description, the information processing system 100 in the present modification acquires the feature groups that have been calculated in the past, are associated with the SOAP note groups and the search purposes, and are stored in the database. Thus, time taken to acquire the search result can be reduced.

### Modification 3 of First Embodiment

In modification 2 of the first embodiment, the feature groups acquired from the feature acquisition apparatus and the feature groups acquired from the database are used in step S1207. In modification 2, the similarity groups with the features corresponding to the search condition data and the similarity groups with the feature groups corresponding to the searched data groups are calculated.

In the present modification, when the database is a vector database, the feature groups acquired from the feature acquisition apparatus are stored in the database, and the searched data groups similar to the search condition data may be identified by using a vector search function provided in the database.

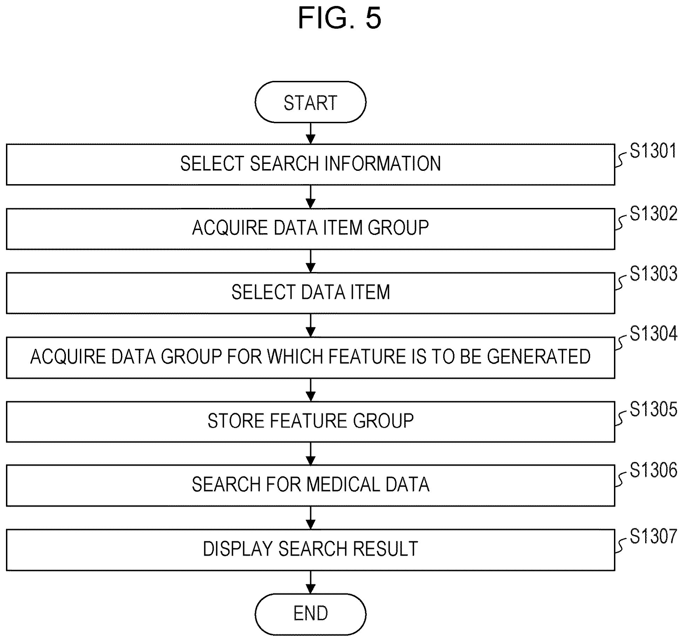

Specifically, an example of a flow of processes executed by the information processing system 100 in the present modification is described with reference to a flowchart illustrated in Fig. 5, corresponding to the flow of the series of processes described in the first embodiment. Order of steps and order of procedures in the steps to be described herein may be changed without causing a contradiction.

In steps S1301 to S1304, processes similar to the processes in steps S101 to S104 of the first embodiment are executed.

In step S1305, a process similar to the process in step S105 of the first embodiment is executed. Furthermore, the feature acquisition function 103c associates the acquired feature groups with the corresponding SOAP notes and the inspection purposes and stores the acquired feature groups in the database.

In step S1306, first, the search function 103e transmits to the database the query in which the feature corresponding to the search condition data is designated as the search condition. The database transmits to the search function 103e, as the search result, the SOAP note groups associated with the feature matching or similar to the feature having been transmitted. At this time, the search results corresponding to the similarity may be sorted by using the sort function provided in the database.

In step S1307, processes similar to the processes in step S108 of the first embodiment are executed.

As has been described, in the present modification, the information processing system 100 acquires the feature groups that have been calculated in the past, are associated with the SOAP note groups and the search purposes, and are stored in the database. Thus, time taken to acquire the search result can be reduced.

### Modification 4 of First Embodiment

The feature acquisition apparatus may include a plurality of encoder groups so as to select and switch the encoder used for calculating the feature based on a single type of input data or a media form based on a single piece of medical data. The encoders exhibit different performance. Each encoder can acquire a comparatively good feature when a type of data suited to the encoder is input. Here, the encoder that can acquire a good feature is characterized as follows: in response to input of data groups the characteristics of which are similar, the encoder outputs feature groups of high similarity; and in response to input of data groups the characteristics of which are dissimilar, the encoder outputs feature groups of low similarity. This difference in performance can be adjusted by, for example, changing the configuration of data sets used for training the encoders and general training conditions for a model architecture, model capacity, hyperparameters, and the like.

Specifically, in the first embodiment, the data item and the template note to be selected based on the search information are varied. Thus, the characteristic of a single text data piece to be generated significantly varies.

To address this, in a process of the present modification corresponding to step S105 of the first embodiment, the feature acquisition function 103c transmits to the feature acquisition apparatus identification information (for example, search information) to identify the characteristic of a single text data piece with the generated single text data piece groups. Based on the received identification information, the feature acquisition apparatus selects and switches the encoder to be used for calculating the feature.

As has been described, in the present modification, the information processing system 100 can acquire the search result in more conformity with the intention in the search by selecting and switching the encoder based on the type of the input data and the media form of a target for which the feature is acquired by the feature acquisition apparatus.

### Modification 5 of First Embodiment

The feature acquisition apparatus may include a plurality of encoder groups so as to select the encoder used for calculating the feature based on the type of each of the plurality of pieces of input data or a media form based on a single piece of medical data. The encoders exhibit different performance. Each encoder can acquire a comparatively good feature when a type of data suited to the encoder is input. Here, meaning of the encoder that can acquire a good feature is similar to that described in modification 4 of the first embodiment. At this time, a plurality of feature groups acquired by inputting a plurality of input data groups into the encoder may be handled as they are or integrated so as to become a single feature. In the case of the integration, the features are joined, added, or subjected to simple averaging, or weighted averaging with a predetermined weight. In the present modification, an example in which the plurality of feature groups are handled as they are without being integrated is described.

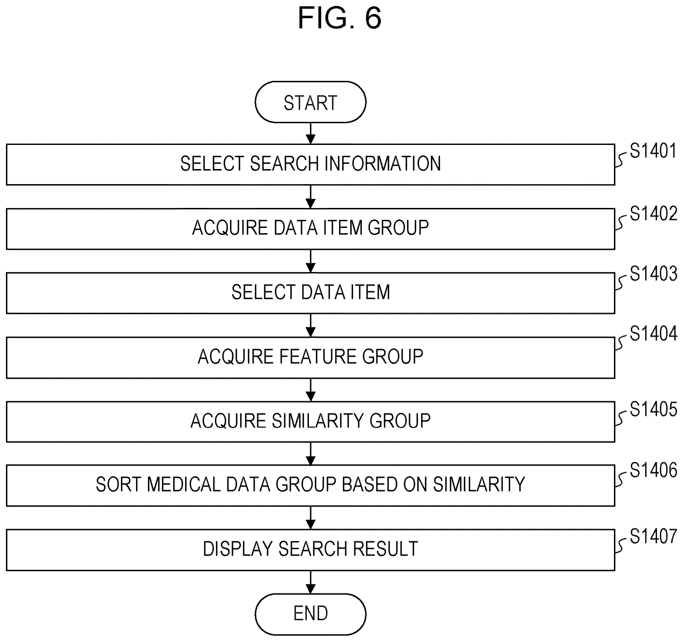

Specifically, an example of a flow of processes executed by the information processing system 100 in the present modification is described with reference to a flowchart illustrated in Fig. 6, corresponding to the flow of the series of processes described in the first embodiment. Order of steps and order of procedures in the steps to be described herein may be changed without causing a contradiction.

In steps S1401 to S1403, processes similar to the processes in steps S101 to S103 of the first embodiment are executed.

In step S1404, the feature acquisition function 103c transmits, to the feature acquisition apparatus (not illustrated), data item combination groups selected in the procedure in step S103. The feature acquisition apparatus inputs each of the data items included in each of the received data item combination groups into any one of the plurality of encoder groups provided in the feature acquisition apparatus to calculate a plurality of feature combination groups and transmit the calculated feature combination groups to the feature acquisition function 103c. The feature acquisition function 103c receives and acquires the plurality of feature combination groups.

In step S1405, the similarity acquisition function 103d uses the plurality of feature combination groups acquired in step S105. The similarity acquisition function 103d calculates the similarity groups between combinations of the features corresponding to the search condition data and each of the feature combination groups corresponding to the searched data groups. In the present modification, the similarity between the combinations of the features is to be calculated. However, when the ith feature in the feature combinations is feature Fi ("i" is the number of selected data items from 1), similarity Si is calculated for features corresponding to the same ordinal number. Specifically, when the search information is "assessment", two data items, "a note about subjective information" and "a note about objective information" are selected. In such a case, a combination of a feature F1 based on "a note about subjective information" and a feature including F2 based on "a note about objective information" is calculated for each SOAP note and acquired. Regarding the similarity, similarity S1 is calculated between a feature F1_Q corresponding to the search condition data and a feature F1_V corresponding to a single piece of the searched data. Furthermore, similarity S2 is calculated between a feature F2_Q corresponding to the search condition data and a feature F2_V corresponding to a single piece of the searched data. A value using the calculated combination of the features corresponding to the search condition data and the combinations of S1 and S2 of the similarity of the combinations of the features corresponding to a single piece of the searched data can be acquired. Furthermore, the combinations of the similarities S1 and S2 may be acquired as a scalar value that can be compared in magnitude with other similarities by, for example, being added to each other or subjected to simple or weighted averaging.

In steps S1406 and S1407, processes similar to the processes in steps S107 to S108 of the first embodiment are executed.

As has been described, in the present modification, the information processing system 100 can acquire the search result in more conformity with the intention in the search by using the plurality of different encoders based on the type of the input data and the media form of the target for which the feature is acquired by the feature acquisition apparatus.

### Modification 6 of First Embodiment

In step S101 of the first embodiment, the search information acquisition function 103b acquires the search information selected by the user. However, the search information may be automatically acquired based on the medical data without the selection operation by the user. That is, characteristically, the search information is determined based on an analysis result of the medical data.

Specifically, description is made with an example in which the user is editing the SOAP note. For example, it is assumed that input of "a note about subjective information" and "a note about objective information" of this SOAP note has been completed or "a note about assessment" is being edited. In this case, conceivably, the user is to complete "a note about assessment" as the next work. Thus, the search information acquisition function 103b automatically acquires "assessment" as the search purpose that is the search information. For example, it is assumed that input of "a note about subjective information", "a note about objective information", and "a note about assessment" of this SOAP note has been completed or "a note about a treatment plan" is being edited. In this case, conceivably, the user is to complete "a note about a treatment plan" as the next work. Thus, the search information acquisition function 103b automatically acquires "planning" as the search purpose that is the search information. For example, it is assumed that input of the entirety of this SOAP note has been completed. In this case, it is difficult to predict what kind of the SOAP note the user wants to refer to. However, it is highly likely that the user wants to refer to a SOAP note in which the whole content (all data items) of the note is similar. Thus, the search information acquisition function 103b automatically acquires "none in particular" as the search purpose that is the search information. After the search information acquisition function 103b has automatically acquired the search information, the search information acquisition function 103b may cause a character string, "assessment" or "planning" to be displayed in the GUI so as to allow the user to refer to the automatically set search information.

Furthermore, after the search information acquisition function 103b automatically acquire the search information, user operation or the like may cause the information processing system 100 to execute the processes in steps S102 and after in the first embodiment to execute the similar data search. Here, the user operation refers to, for example, pressing down a button control intended to represent "Start similar data search" in the GUI.

When the automatically acquired search information does not match the user's search intention, the user may perform the operation described in step S101 of the first embodiment, and subsequently the procedures of the present modification may shift to the flow of a series of the processes of the first embodiment.

As another example, when the medical data is, for example, the medical record information, the search information acquisition function 103b may automatically acquire the search information based on the diagnosis recorded in the medical record information. In this case, for example, the search information is a character string based on the diagnosis such as "breast cancer" or "lung cancer". Before the whole medical record information stored in the electronic medical records is acquired in step S102 of the first embodiment, only the medical record articles in which the diagnosis is recorded may be acquired. Then, in step S103 of the first embodiment, the feature acquisition function 103c selects data items useful for diagnosis of diseases corresponding to the search information. For example, when the search information is "breast cancer", data relating to the presence/absence of family disease history in category form and a mammographic image in image form are selected as the data items. Then, in step S104 of the first embodiment, the feature acquisition function 103c processes the data items according to need so as to allow the data items to be input into various types of encoders provided in the feature acquisition apparatus. For example, the data relating to the presence/absence of family disease history is converted into data in text form such as "Mother has medical history of breast cancer" by using a rule-based process or the like so as to allow the data to be input into a text encoder. For example, the mammographic image is converted into pixel value group data so as to allow the image to be input into an image encoder. The subsequent steps can be processed by the procedures in step S1405 and after of modification 5 of the first embodiment that execute the similar data search in consideration of the similarity relating to a plurality of data item groups.

As yet another example, when the medical data is, for example, the medical record information, the search information acquisition function 103b acquires the CT image based on the link information to the inspection data recorded in the medical record information. The search information acquisition function 103b may automatically acquire the search information based on a result of an image analysis of the CT image. In this case, for example, the search information is a character string such as "lung cancer" or "liver cancer". Then, in step S103 of the first embodiment, the feature acquisition function 103c selects data items useful for diagnosis of diseases corresponding to the search information. For example, when the search information is "lung cancer", data relating to the presence/absence of smoking history in category form and the CT image in image form are selected as the data items. Then, in step S104 of the first embodiment, the feature acquisition function 103c processes the data items according to need so as to allow the data items to be input into various encoders provided in the feature acquisition apparatus. For example, the data relating to the smoking history is converted into data in text form such as "Having smoking history; Brinkman Index is 40 × 20 = 800" by using a rule-based process or the like so as to allow the data to be input into a text encoder. For example, the CT image, which can be input into the image encoder as it is, is not processed. The subsequent steps can be processed by the procedures in step S1405 and after of modification 5 of the first embodiment that execute the similar data search in consideration of the similarity relating to a plurality of data item groups.

As has been described, in the present modification, by analyzing the state of the medical data, the information processing system 100 can automatically acquire the search information and execute the similar data search.

### Modification 7 of First Embodiment

In step S104 of the first embodiment, by the processing procedure for the data items using the template note, the feature acquisition function 103c generates the data in text so as to allow the feature acquisition apparatus to input the data into the encoder. In this processing procedure for the data items, the information processing system 100 may add a predetermined character string (text). This procedure of adding a character string may be executed regardless of whether the feature acquisition function 103c utilizes the template note.

For example, when the search information is "assessment", a character string such as "examination in consideration of the above-described information" may be added. For example, when the search information is "planning", a character string such as "Things to do hereafter in the above-described situation" may be added.

The character string to be added may be determined based on information other than the search information. For example, a result or the like of processing of the data items included in the medical data by using a predetermined analytical process. Specifically, when a predetermined disease X is positive as a result of the analytical process of blood inspection and image inspection included in the medical data, a character string such as "Note that disease X is positive" may be added.

The character string to be added may be a predetermined character string recorded in the information processing system 100.

As has been described, in the present modification, when the feature acquisition apparatus further processes the data in text form so as to input the data into the encoder, the information processing system 100 can guide the trend of the feature calculated by the encoder. That is, a characteristic of medical data is emphasized in a feature, and a feature becoming noise that degrades the search accuracy in the similar data search is reduced. This reduces the likelihood of including medical data having low relevance to the user's search intention.

### Second Embodiment

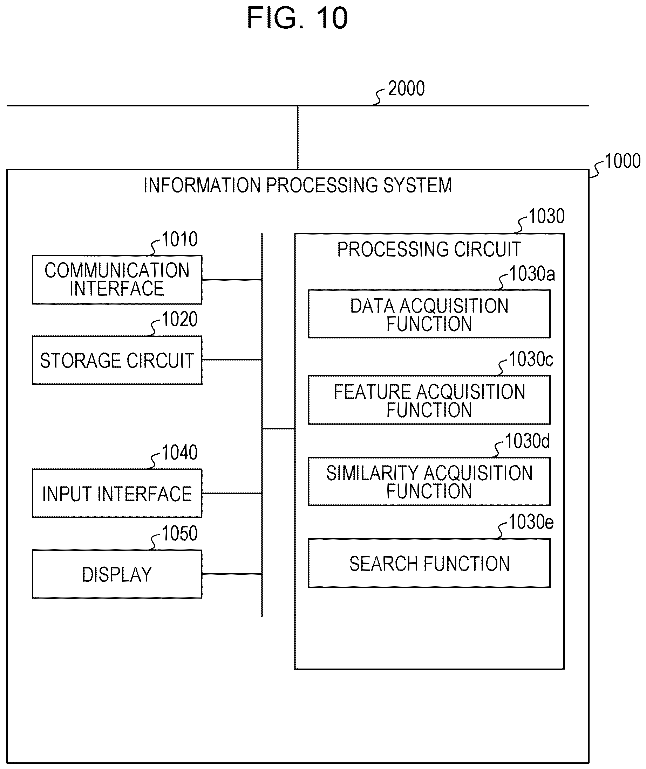

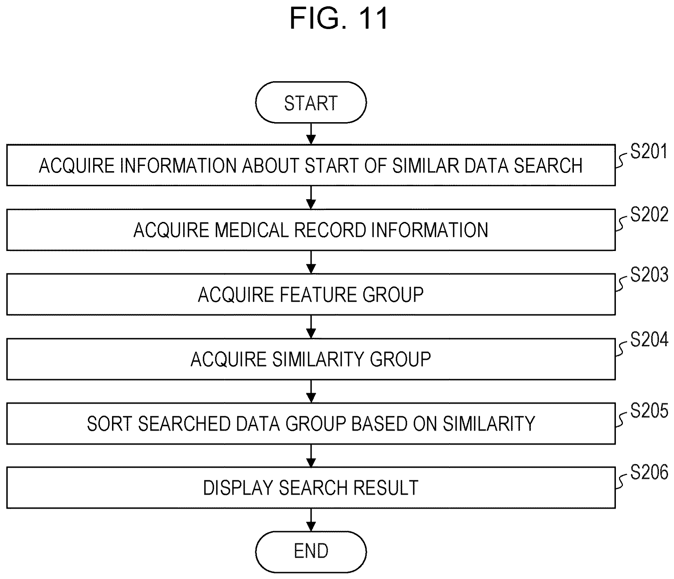

Hereinafter, in a second embodiment, a search process of medical data similar to medical data including a plurality of types of data executed by an information processing system 1000 is described with reference to Figs. 10 and 11. Here, the plurality of types of data refer to data including data items having different media forms. In the present embodiment, for ease of description, the medical data is the inspection data of the patient in the medical field. Specifically, the medical data is the medical record information of the electronic medical record. Furthermore, the medical data management apparatus is an electronic medical record system that is a database managing the medical record information.

Referring to Fig. 10, the functional configuration of the information processing system 1000 according to the present embodiment is described. The information processing system 1000 includes a communication interface 1010, a storage circuit 1020, a processing circuit 1030, an input interface 1040, and a display 1050. The information processing system 1000 can be communicably connected to the network 2000 via the communication interface 1010. Functional configurations of the communication interface 1010, the storage circuit 1020, the input interface 1040, and the display 1050 are similar to those in the first embodiment. Thus, description of these component is omitted.

The processing circuit 1030 according to the present embodiment includes a data acquisition function 1030a that realizes a data acquisition unit configured to acquire the medical data and a feature acquisition function 1030c that realizes a feature acquisition unit configured to acquire the feature. The processing circuit 1030 also includes a similarity acquisition function 1030d that realizes a similarity acquisition unit configured to acquire the similarity between pieces of medical data. The processing circuit 1003 also includes a search function 1030e that realizes a search unit configured to acquire a search result of similar medical data.

When the information processing system 1000 has the above-described functional configuration, the information processing system 100 can adequately calculate the similarity to the medical data including different types of data. Medical data (the second medical data) similar to the first medical data can be acquired with high accuracy by using the similarity.

Here, for example, each of the processing functions realized by the corresponding elements of the processing circuit 1030 illustrated in Fig. 10 is store in the storage circuit 1020 in the form of a program executable by the computer. The elements include the data acquisition function 1030a, the feature acquisition function 1030c, the similarity acquisition function 1030d, and the search function 1030e.

The processing circuit 1030 reads the programs from the storage circuit 1020 and executes the read programs, thereby realizing the functions corresponding to the programs. That is, the processing circuit 1030 having read the programs has the functions realized by the data acquisition function 1030a, the feature acquisition function 1030c, the similarity acquisition function 1030d, and the search function 1030e.

Specifically, the information processing system 1000 according to the present embodiment has the data acquisition function 1030a configured to acquire first medical data that relates to the subject and includes the different types of data. The information processing system 1000 has the feature acquisition function 1030c configured to represent the characteristic of the medical data. The feature acquisition function 1030c is characterized in that the feature acquisition function 1030c is configured to acquire at least a subset of the features by inputting medical data including the different types of data included in the first medical data into the encoder. Here, the encoder is at least one of the encoder of the large language model and the encoder of the large multimodal model. In addition, the information processing system 1000 has the similarity acquisition function 1030d configured to acquire the similarity between the feature and the feature of another piece of medical data. The information processing system 1000 also has the search function 1030e configured to acquire the search result of the medical data (the second medical data) similar to the first medical data based on the similarity. With the above-described configuration, adequate similarity can be calculated for the medical data including the different types of data, and, by using such similarity, the search result with high accuracy can be acquired. Hereinafter, an example of the flow of the processes executed by the information processing system 1000 according to the present embodiment is described. Order of steps and order of procedures in the steps to be described herein may be changed without causing a contradiction.

In the following description, for ease of understanding, it is assumed that, currently, the user operates the input interface 104 to select the patient's medical record information from the electronic medical record system, review the selected information, and use it to create a treatment plan. It is also assumed that the user is performing the similar data search on the electronic medical record system to search for, for reference, other medical record information similar to the medical record information. In the following description, the selected medical record information is referred to as "search condition data", and the other medical record information is referred to as "searched data".

In step S201, the data acquisition function 1030a acquires information about the start of inspection of similar data similar to the first medical data. The user operates the GUI displayed by the display 105 to instruct the start of inspection. The operation refers to, for example, pressing down a button control intended to represent "Start similar data search" in the GUI.

In step S202, the data acquisition function 1030a acquires all the medical record information stored in the electronic medical record. The medical record information is medical data including data items in various media forms such as the numerical form, the text form, and the image form.

In step S203, the feature acquisition function 1030c transmits, to the feature acquisition apparatus (not illustrated), data item combination groups included in the medical record information acquired in step S202. The feature acquisition apparatus inputs each of the data items included in the received data item combination groups into various types of encoders provided in the feature acquisition apparatus and transmits the calculated feature combination groups to the feature acquisition function 1030c. The feature acquisition function 1030c receives and acquires the feature groups.

The various types of encoders include a numerical value encoder into which data in numerical form can be input, a text encoder into which data in text form can be input, and an image encoder into which data in image form can be input. In other words, data items in various types of media forms included in the medical record information are input into the various types of encoders, and the various types of encoders calculate the features.

In step S204, the similarity acquisition function 1030d uses the feature combination groups acquired in step S203. The similarity acquisition function 1030d calculates the similarity groups between combinations of the features corresponding to the search condition data and each of the feature combination groups corresponding to the searched data groups. Here, each of the similarities can be calculated by the method described in step S1406 of modification 5 of the first embodiment.

In step S205, the search function 1030e sorts the searched data groups corresponding to the similarity based on the magnitude of the values of the similarity calculated in step S204. When the above-described processing is performed, even when a large amount of searched data exists, accessibility, for the user, of the medical record information similar to the search condition data is improved.

In step S206, the search function 1030e causes the sorted searched data groups to be displayed as the search result in the GUI of the display 105.

From the above description, the information processing system 1000 according to the present embodiment can search for the medical data (the second medical data) similar to the first medical data including the plurality of types of data.

With the technique of the present disclosure, similarity can be appropriately calculated.

While the present disclosure has been described with reference to embodiments, it is to be understood that the present disclosure is not limited to the disclosed embodiments, but is defined by the scope of the following claims.

Various embodiments have been described in detail above but it will be understood that the present disclosure is not limited to these embodiments and encompasses all modifications, variants, alternatives and equivalents falling within the scope of the appended claims.

## Claims

1. An information processing system (100) comprising:
a data acquisition unit (103a) configured to acquire first medical data including a plurality of items or different types of data relating to a subject;
a feature acquisition unit (103c) configured to acquire features representing characteristics of medical data, the feature acquisition unit (103c) acquiring at least a subset of the features by inputting the medical data included in the first medical data into at least one of an encoder of a large language model and an encoder of a large multimodal model; and
a similarity acquisition unit (103d) configured to acquire similarity between the features and a feature of another piece of the medical data.

2. The information processing system (100) according to claim 1,
wherein the first medical data includes the plurality of items,
wherein the information processing system further includes a search information acquisition unit (103b) configured to acquire search information for searching second medical data similar to the first medical data, and
wherein the feature acquisition unit (103c) is configured to acquire at least a subset of the features based on the search information.

3. The information processing system (100) according to claim 1 or 2, further comprising:
a search unit (103e) configured to acquire a search result of second medical data similar to the first medical data based on the similarity.

4. The information processing system (100) according to claim 1,
wherein the first medical data includes the different types of data,
wherein the information processing system further includes a search information acquisition unit (103b) configured to acquire search information for searching second medical data similar to the first medical data, and
wherein the feature acquisition unit (103c) is configured to acquire the features based on the search information.

5. The information processing system (100) according to claim 2, or claim 3 in combination with claim 2,
wherein the search information includes user's search intention, and the search information changes the features acquired by using the similarity acquisition unit (103d) via at least one of the encoder of the large language model and the encoder of the large multimodal model.

6. The information processing system (100) according to claim 4,
wherein the search information includes user's search intention, and the search information changes the features acquired by using the similarity acquisition unit (103d) via at least one of the encoder of the large language model and the encoder of the large multimodal model.

7. The information processing system (100) according to claim 2, or claim 3 in combination with claim 2,
wherein the search information includes user's search intention, and the search information acquires the features from first features acquired via at least one of the encoder of the large language model and the encoder of the large multimodal model by using the similarity acquisition unit (103d).

8. The information processing system (100) according to claim 4,
wherein the search information includes user's search intention, and the search information acquires the features from first features acquired via at least one of the encoder of the large language model and the encoder of the large multimodal model by using the similarity acquisition unit (103d).

9. The information processing system (100) according to claim 2, or claim 3 in combination with claim 2,
wherein the feature acquisition unit (103c) is configured to input a prompt including user's search intention into the encoder for acquiring the feature in conformity with the user's search intention.

10. The information processing system (100) according to claim 4,
wherein the feature acquisition unit (103c) is configured to input a prompt including user's search intention into the encoder for acquiring the feature in conformity with the user's search intention.

11. The information processing system (100) according to any one of the preceding claims, when dependent upon claim 2 or 4,
wherein the search information is set based on user's instruction.

12. The information processing system (100) according to any one of the preceding claims, when dependent upon claim 2 or 4,
wherein the search information is determined based on an analysis result of the medical data.

13. The information processing system (100) according to any one of the preceding claims,
wherein the encoder is trained such that the similarity of feature groups to be output in response to input of medical data groups in which characteristics of predetermined data item groups are similar is higher than the similarity of feature groups to be output in response to input of medical data groups in which characteristics of the predetermined data item groups are dissimilar.

14. The information processing system (100) according to any one of the preceding claims, when dependent upon claim 3,
wherein the search unit (103e) is configured to cause the search result to be displayed by a display unit (105).

15. The information processing system (100) according to any one of the preceding claims, when dependent upon claim 3,
wherein the search unit (103e) is configured to acquire the search result of the second medical data based on the similarity and values of one or more items included in the medical data.

16. The information processing system (100) according to any one of the preceding claims, when dependent upon claim 2,
wherein at least one of the large language model and the large multimodal model includes a plurality of encoders, and
wherein the feature acquisition unit (103c) is configured to select the encoder based on the search information.

17. The information processing system (100) according to any one of the preceding claims, when dependent upon claim 2,
wherein at least one of the large language model and the large multimodal model includes a plurality of encoders, and
wherein the feature acquisition unit (103c) is configured to select the plurality of encoders based on the search information.

18. The information processing system (100) according to claim 17,
wherein the feature acquisition unit (103c) is configured to acquire the features by integrating the features acquired by using the plurality of selected encoders.

19. A method for processing information, the method comprising:
acquiring first medical data including a plurality of items or different types of data relating to a subject;
acquiring features representing characteristics of medical data, the acquiring features acquiring at least a subset of the features by inputting the medical data included in the first medical data into at least one of an encoder of a large language model and an encoder of a large multimodal model; and
acquiring similarity between the features and a feature of another piece of the medical data.

20. The method according to claim 19,
wherein the first medical data includes the plurality of items,
wherein the method further includes acquiring search information for searching second medical data similar to the first medical data, and
wherein, in the acquiring features, at least a subset of the features are acquired based on the search information.
